Europäisches Patentamt

European Patent Office

Office européen des brevets

(11) Publication number: **0 501 320 A1**

## EUROPEAN PATENT APPLICATION

(21) Application number: **92102897.3**

(22) Date of filing: **21.02.92**

(51) Int. Cl.⁵: **C07K 15/00**, A61K 39/395

(30) Priority: **26.02.91 JP 30749/91**
**26.08.91 JP 213452/91**

(43) Date of publication of application:
**02.09.92 Bulletin 92/36**

(84) Designated Contracting States:
**AT BE CH DE DK ES FR GB GR IT LI LU MC NL PT SE**

(71) Applicant: **Hirasawa, Keisuke**
**12-11 Nakajuku**
**Itabashi-ku, Tokyo-to(JP)**

(72) Inventor: **Hirasawa, Keisuke**
**12-11 Nakajuku**
**Itabashi-ku, Tokyo-to(JP)**

(74) Representative: **Türk, Dietmar, Dr. rer. nat.**
**Türk, Gille + Hrabal Patentanwälte**
**Brucknerstrasse 20**
**W-4000 Düsseldorf 13(DE)**

(54) Immunopotentiating drug containing an anti-testosterone antibody.

(57) An immunopotentiating drug comprising an anti-testosterone antibody as an effective ingredient. The drug of the present invention has a clear immunopotentiating activity and does not have serious side effects or toxicity.

EP 0 501 320 A1

The present invention relates to an immunopotentiating drug.

Although some immunopotentiating drugs have been introduced for the treatments of infectious diseases caused by viruses or bacteria and of cancer chemotherapy, the effectivenesses are insufficient to cure those diseases. Partly because immune systems of living animals are complicated.

So, a novel immunopotentiating drug which does not have serious side effects or toxicity has been expected.

An immunopotentiating drug comprising an anti-testosterone antibody of the present invention has been discovered from a series of studies on important questions, (1) why males are lower resistant to bacteria and viruses such as human immunodeficiency (AIDS) virus and hepatitis B type virus infections than females (ref. Wheater DWF, Hurst EW, J. Pathol. Bact. The effect of sex on bacterial infections and on the chemotherapy of one of them. Vol. 82, 117-130, 1961) (ref. London WT, Drew JS, Proc. Natl. Acad. Sci., Sex differences in hepatitis B infection among patients receiving chronic dialysis treatment., Vol. 74, pp2561-2563, 1977), (2) why males produce lower titer of antibody against an incompatible tumour than females, which leads to that males suffer from cancers such as leukemia and prostate cancer more than females (ref. Batchelor JR, Chapman BA, Immunology. The influence of sex upon the antibody response to an incompatible tumour. Vol. 9, 553- 564, 1965) and (3) why male recipients accept allogeneic organ graft longer than females in both clinical and experimental cases (ref. Terasaki PI, Mickey MR, Cecka M, Cicciarelli J, Cook D, Iwaki Y, Toyotome A, Wang L, Clin. Transplant., Overview, Vol. 3, pp465-490, 1987) (ref. Hirasawa K, Enosawa S, Transplant. Proc. Sex differences in the prolongation of allogeneic skin graft survival in rats treated with cyclosporine A: Effects of orchiectomy and pregnancy. Vol. 21, pp881-884, 1989).

Previous studies suggested that the sex associated differences may be attributed to sex hormones shown by that exogenous testosterone could be immunosuppressive (ref. Hirasawa K, Enosawa S., Transplantation, Effects of sex steroid hormones on sex-associated differences in the survival time of allogeneic skin graft in rats. Vol. 50, pp639-641, 1990). However, as to endogenous testosterone, an action on immune system in hosts is not at all known hitherto.

An object of the present invention is to provide an immunopotentiating drug having no serious side effects.

This and other object of the present invention will become apparent from the description hereinafter.

It has now been found not only that endogenous testosterone works immunosuppressively in hosts, but also that anti-testosterone antibody surprisingly possesses immunopotentiating activity in long periods without obvious side effects in terms of reversing immunosuppressive activity of the non-specific immunosuppressive drug such as Cyclosporine and of total body irradiation. That is, it has been found that anti-testosterone antibodies possess potentiating activity to fundamental immunity of hosts. According to the present invention, there is provided an immunopotentiating drug comprising an anti-testosterone antibody as an effective ingredient.

Figure 1 is a graph showing effects of various dosages of an anti-testosterone monoclonal antibody on the graft survival time in recipients treated with non-specific immunosuppressive drug, Cyclosporine in Example 2.

Figure 2 is a graph showing the relationship between percentage of the graft survival and days after the operation in Example 2.

Anti-testosterone antibodies which are active ingredients of the present invention can be prepared by any usual methods. The antibodies can be antisera (polyclonal antibodies), monoclonal antibodies against testosterone or the like including anti-idiotype.

For example, antisera, polyclonal antibodies against testosterone are taken from sera of animals such as rats immunized with bovine serum albumin conjugated testosterone-3-(carboxymethyl oxime). For example, monoclonal antibodies against testosterone are produced from clones made by hybridization of non-immunogloblin-producing myeloma cells of mice or the like and spleen cells of the animals immunized as above.

The immunopotentiating drug of the present invention can be administered by parenteral administration such as intravenous administration, subcutaneous administration or intramuscular administration. The administration route is not particularly limited and can be suitably selected according to the purpose of the treatments.

The immunopotentiating drug of the present invention can be used in various preparation forms such as injection, transfusion solution and eye drops containing about 0.1 to about 3 % by weight of anti-testosterone antibody. These various preparations can be prepared in usual methods by using conventional additives which are generally usable in the field of pharmaceutical preparation such as excipient, binder, solvent, solubilizer, emulsifier or suspending agent, according to the purpose of the treatment.

As such additives, there are, for example, lactose, sucrose, glucose, starch, crystalline cellulose, hydroxypropylcellulose, carboxymethylcellulose, arabic gum, gelatin, magnesium alumino meta silicate, anhydrous calcium phosphate, citric acid, trisodium citrate, hydroxypropylcellulose, sorbitol, sorbitan esters of fatty acids, polyvinylpyrrolidone, vegetable oil such as peanut oil or olive oil, benzyl alcohol, propylene glycol, water and the like.

When the immunopotentiating drug of the present invention is used as an injection solution, an effective dosage is chosen from a range of 0.1-50 mg of anti-testosterone monoclonal antibody / day as an effective ingredient for adults.

Since the immunopotentiating drug of the present invention comprising an anti-testosterone antibody as an active ingredient potentiates the immunity of the hosts which are immunosuppressed by non-specific immunosuppressive drugs such as Cyclosporine or treatment of total body irradiation, the immunopotentiating drug of the present invention is useful for raising the resistance against invaders such as viruses and bacteria, and tumors. The immunopotentiating drug of the present invention is, therefore, useful for treatments of infectious diseases caused by human immunodeficiency (AIDS) virus, hepatitis B virus, influenza virus or gram positive or negative bacteria and chemotherapies for cancers such as prostate cancer and leukemia. The effectiveness of the drug of the present invention is potentiation of fundamental immunity of patients, i.e. potentiation of recognition of not-self substances and foreign antigens, potentiation of sensitization, potentiation of T-cell growth and the like.

The immunopotentiating drug of the present invention is more specifically described and explained by means of the following Examples. It is to be understood that the present invention is not limited to such Examples, and various changes and modifications can be made in the invention without departing from the spirit and scope thereof.

Reference Example

Preparation of anti-testosterone monoclonal antibody

Anti-testosterone monoclonal antibody (rat origin clone No. F2) in ascites of rats was purchased from Intra Pharm laboratories Ltd., Israel (ref. Kohen P, Lichter S, Serono Symposia Publications, Monoclonal antibodies to steroid hormones, Vol 30, pp87-95, 1986). The ascites were centrifuged and diluted ten times with 15 mM, pH 7.0 phosphate buffer solution. Then the solution of the ascites was applied on the hydroxylapatite column ($0.7~cm^2$ x 20 cm) equilibrated with 15 mM, pH 7.0 phosphate buffer solution. The elution procedures were carried out by stepwise elutions with from 15 mM to 300 mM phosphate buffer solutions as described previously (ref. Bukovsky J, Kennett RH, Hybridoma, Simple and rapid purification of monoclonal antibodies from cell culture supernatants and ascites fluids by hydroxylapatite chromatography on analytical and preparative scales. Vol 6, pp219-228, 1987). Then the biochemical and immunological characterizations of thus purified antibodies were determined as follows:

Anti-testosterone monoclonal antibody

Specification

    (1) Specific activity:    460 pico moles testosterone/mg protein
    (2) Antibody Isotype:    $IgG_1$
    (3) Cross reactivity:

| Steroids | Cross reactivity (%) |
|---|---|
| Testosterone | 100 |
| 5-$\alpha$ -Dihydrotestosterone | 2.00 |
| 5-$\beta$ -Dihydrotestosterone | 15.00 |
| Androsterone | 4.00 |
| Progesterone | <0.1 |
| Cortisol | <0.1 |
| Estradiol | <0.1 |

Example 1

Preparation of injection solution

The injection solution of the anti-testosterone monoclonal antibody was prepared as the following formulation.

| Anti-testosterone monoclonal antibody | 4.5 mg |
|---|---|
| Saline | balance |
| total | 1 mℓ |

The treatment of the antibody can be carried out within six hours after the operation as a single administration.

Example 2

Effect of anti-testosterone monoclonal antibody on the survival time of the skin graft (1)

The skin graftings were performed between fully allogeneic inbred rat strains, DA-RT1a rats (donor) and PVG-RT1c rats (recipient), which were purchased from Harlan Olac Ltd., U.K. Sexually mature adult male rats, 12-15 weeks old, (body weight, 250-290 g) were used for the experiments. Six recipients were used in each group.

In order to show that anti-testosterone monoclonal antibody has an immunopotentiating activity, attempt was made for the antibody to antagonize the non-specific immunosuppressive activity induced by Cyclosporine which inhibits lymphocyte blast formation stimulated by foreign antigens, lectins or the like.

Full thickness of skin graft (3 x 3 cm) from ventral trunk of adult male DA-RT1a rats were grafted in adult male PVG-RT1c rats according to the usual method (ref. Towpic E, Kupiec-Weglinski JW, Schneider TM, Tyler D, Padberg W, Araneda D, Tilney NL, Transplantation, Cyclosporine and experimental skin allograft.II. In definite survival and development of specific immunogic unresponsiveness, Vol. 40, pp714-718, 1985). The rejection of the skin grafts were determined as total epithelial necrosis.

Cyclosporine (trade name: Sandimmun, Sandoz, Basel, Switzerland) as a non-specific immunosuppressive drug was administered intramuscularly at a dose of 20 mg/kg/day into a rear leg within six hours after the operation and thereafter every 24 hour for two weeks.

As an anti-testosterone monoclonal antibody, the antibody obtained in Reference Example was used here.

The effect of the anti-testosterone monoclonal antibody was examined by the comparison of the survival times of the skin grafts in the recipients differently treated as follows:

| Groups: | Post-operative treatments (Pre-operative treatments can be also done.) |
|---|---|
| A group: | Cyclosporine only |
| B group: | Cyclosporine + the antibody (0.10 mg in 1 mℓ of saline). |
| C group: | Cyclosporine + the antibody (0.75 mg in 1 mℓ of saline). |
| D group: | Cyclosporine + the antibody (1.50 mg in 1 mℓ of saline). |
| E group: | Cyclosporine + the antibody (4.50 mg in 1 mℓ of saline). |
| F group: | Olive oil (1 mℓ /mg/day) + 1 mℓ of saline |

The administrations of the solution of the antibody (1 mℓ /rat) were carried out on the day of the operation only.

The recipients in F group were treated with olive oil and saline in place of Cyclosporine and the antibody, respectively.

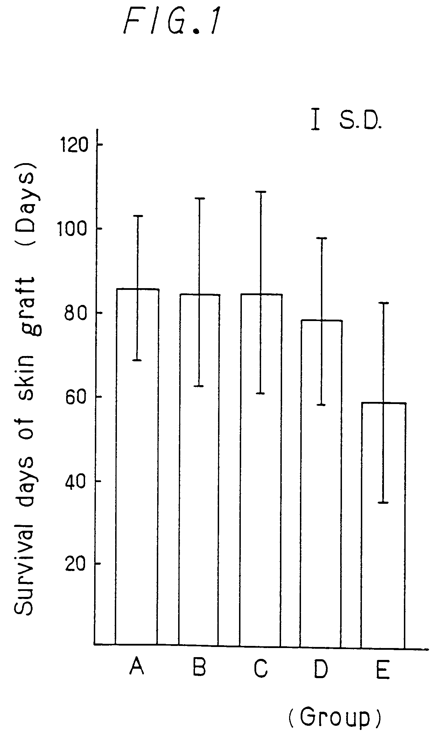

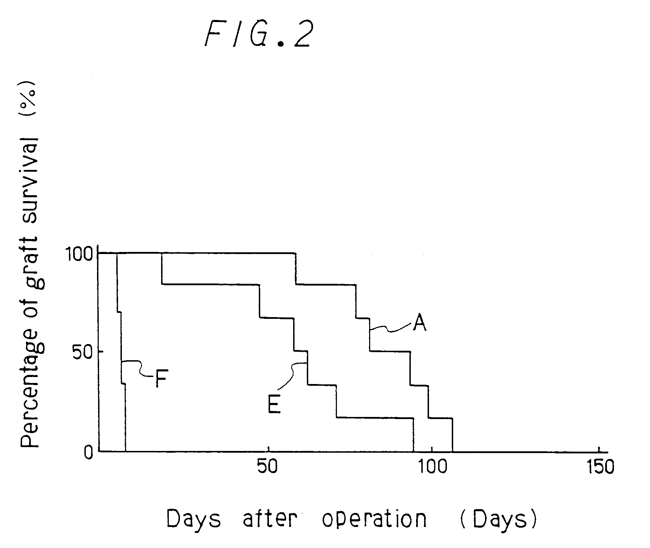

The results are shown in Figures 1 and 2.

The PVG-RT1c recipients in non-Cyclosporine treated F group acutely rejected DA-RT1a skins on the 7-9th day after the day of the operation. On the other hand, the survival time of the grafts in the recipients treated with only Cyclosporine (A group) was 85.8±17.0 days (mean survival day ± SD, hereinafter the same). However, as shown in Figure 1, the survival time of the skin grafts in the recipients administered with both Cyclosporine and the anti-testosterone monoclonal antibody (B-E groups) was reduced in the antibody dose dependent manner. In particular, the survival time was significantly reduced when administered with at least 1.5 mg of the antibody (D and E groups) in comparison with that in A group. As shown in Figure 2, an inhibitory effect of the antibody on the graft survival times is clear between A and E groups. The percentage of the graft survival on the same day after the operation was significantly reduced in E group compared with A group. The difference in the graft survival times between A and E groups was statistically significant at p = 0.0009 under the Student T test.

The results stated here showed that anti testosterone monoclonal antibody potentiated immune responses of hosts which were immunosuppressed by the non-specific immunosuppressive drug, Cyclosporine. Therefore, it was proved that the antibody had non-specific immunopotentiating activity.

Example 3

Effect of anti-testerone monoclonal antibody on the survival time of the skin graft (2)

In the Example 3, another non-specific immunosuppressive treatment, total body irradiation was adopted on recipients in order to clarify that immunopotentiating activity of the anti-testosterone monoclonal antibody is not unique to only hosts treated with a non-specific immunosuppressive drug, Cyclosporine. It has been known that the treatment of total body irradiation makes hosts immunosuppressive by reducing the number of white blood cells. The irradiation was carried out on the recipients at 750 rads/rat for three hours prior to the skin grafting. Other experimental materials and procedures were the same as in Example 2.

The PVG-RT1c adult male recepients in a group not treated with total body irradiation acutely rejected DA-RT1a adult male skins on the 7-8th day after the day of the operation. On the other hand, the survival time of the grafts in the recipients immunosuppressed by total body irradiation was 17.2±1.5 days. When the total body irradiated six resipients was administered with the anti-testosterone monoclonal antibody (4.5 mg in 1 mℓ saline) as a single administration whithin six hours after the operation, the survival time of the grafts was reduced to 14.0±0.9 days. The difference in the graft survival times between the group not administered with anti-testosterone antibody and the group administered with anti-testosterone antibody was statistically significant at p = 0.0001 under the Student T test. Therefore, the anti-testosterone monoclonal antibody was proved to potentiate fundamental immunity of the hosts by reversing the immunosuppressive effect of the irradiation which decreases a number of white blood cells.

Example 4

Acute toxicity test

Male PVG-RT1c rats (8 weeks old, body weight 200-220 g) were administered intravenously with the injection solution of the anti-testosterone monoclonal antibody obtained in Reference Example (10 mg/mℓ of saline). The values of the $LD_{50}$ were not less than 40 mg/ kg.

In addition to the ingredients used in the Examples, other ingredients can be used in the Examples as set forth in the specification to obtain substantially the same results.

**Claims**

1. An immunopotentiating drug comprising an anti-testosterone antibody as an effective ingredient.

2. The drug of claim 1 comprising an anti-testosterone antibody and pharmaceutically usable carrier or additive.

3. Use of an anti-testosterone antibody as an effective ingredient for potentiating fundamental immune responses.

4. Use of an anti-testosterone antibody for manufacture of medicament for potentiating fundamental immune responses.

# FIG.1

FIG.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | MEDLINE Abstract number: 91118507 HIRASAWA K. et al:"Sex-associated differences in organ transplantation: different effects of steroid hormones, testosterone, estradiol progesterone, and prednisolone on the survival time off allogeneic skin graft in rats treated with cyclosporine A." & TRANSPLANT PROC., February 1991, 23 , p 714-715. | 1-4 | C07K15/00 A61K39/395 |
| X D | MEDLINE Abstract number: 91020368 HIRASAWA K. et al: " Effects of sex steroid hormones on sex-associated differences in the survival time of allogeneic skin grafts in rats" Evidence that testosterone enhances and estradiol reverses the immunosuppressive activity of cyclosporine. & TRANSPLANTATION, October 1990, 50 (4), p 637-41 | 1-4 | |
| X | MEDLINE Abstract number: 89326493 CARLSTEN H. et al: "Oestradiol suppression of delayed-type hypersensitivity in autoimmune (NZB/NZW) F1 mice is a trait inherited from the healthy NZW parental strain." & IMMUNOLOGY , June 1989, 67(2), p 205-9 | 1-4 | TECHNICAL FIELDS SEARCHED (Int. Cl.5) A61K G01N C07K |
| A | EP-A-0 226 985 (BOEHRINGER MANNHEIM) * the whole document * | 1-4 | |
| A | US-A-4 197 286 (PEMMARAJU N. RAO) * the whole document * | 1-4 | |

The present search report has been drawn up for all claims

| Place of search | Date of completion of the search | Examiner |
|---|---|---|
| BERLIN | 06 MAY 1992 | AVEDIKIAN P.F. |